# EUROPEAN PATENT APPLICATION

(11) **EP 2 325 297 A2**
(43) Date of publication of application: **25.05.2011**
(21) Application number: 10380136.1
(22) Date of filing: 28.10.2010
(51) Int. Cl.: C12N 5/0789, C12M 3/02, A61K 35/14

(54) **Procedure for the undifferentiated or myeloid lineage biased expansion of haematopoietic stem cells from umbilical cord blood, mobilized peripheral blood or bone marrow**

(30) Priority: 02.11.2009 ES 200930939
(71) Applicant: Banc De Sang I Teixits, E-08035 Barcelona (ES)
(72) Inventor: Garcia Lopez, Joan, 08029 Barcelona (ES); Casamayor Genesca, Alba, 08230 Matadepera Barcelona (ES); Cairo Badillo, Jordi Joan, 08202 Sabadell Barcelona (ES); Pla Calvet, Arnau, 08173 Sant Cugat del Valles Barcelona (ES)
(74) Representative: Durán Moya, Carlos

(57) **Abstract**

Procedure for the undifferentiated or myeloid lineage biased expansion of haematopoietic stem cells coming from umbilical cord blood, mobilized peripheral blood or bone marrow.

Procedure for the undifferentiated or myeloid lineage biased expansion of haematopoietic stem cells (HSCs). More specifically, the present invention relates to a procedure of expansion of HSCs from umbilical cord blood, bone marrow or mobilized peripheral blood. Said procedure comprises the steps of expansion culturing of the purified CD34+ cells at constant volume, expansion culturing of said cells at variable volume and the conditioning of the CD34+ cells for transplantation. With this procedure the dose of undifferentiated or myeloid lineage biased HSCs which is necessary for their clinical use is produced reproducibly, robustly and safely.

## Description

### DESCRIPTION

The present invention relates to a procedure applicable to the in vitro undifferentiated or myeloid lineage biased expansion of haematopoietic stem cells (HSCs). Said procedure comprises the steps of expansion of the purified stem cells at constant volume, expansion of said cells at variable volume, and the conditioning of the cells for transplantation. With this procedure it is possible to expand the HSCs reproducibly and robustly until clinically significant doses are obtained, rapidly and safely, either of immature cells or cells biased to myeloid lineage.

HSCs are multi-potent cells capable, by means of a complex process of self-renewal and differentiation, of maintaining the homeostasis of the haematopoietic organ, giving rise, in precisely regulated form, to all the cell lineages of the blood. These lineages are organized into two large groups with distinct functions: myeloids and lymphoids. The myeloids are grouped into: monocytes, macrophages, neutrophils, basophils, and eosinophils, which combat infections in the organism; the megakaryocytes (platelets), which take part in the blood-clotting process, and also erythrocytes, which transport oxygen to the tissues. The other large group of blood cells are the lymphoids, composed of: B cells, T and NK cells, which are concerned with immunological vigilance.

The autologous or allogeneic transplantation of HSCs represents an important therapeutic alternative for haematological diseases such as neoplasia, primary immune deficiencies and metabolic disorders. The principal sources for the therapeutic transplantation of HSCs are: bone marrow (BM), mobilized peripheral blood (MPB) and umbilical cord blood (UCB). Historically, bone marrow represented the principal source of stem cells for transplantation in paediatric and adult patients. However, the difficulty of finding compatible donors and the risk of suffering from graft-versus-host disease, associated with allogeneic transplantation, has limited its applicability. A problem which BM shares with mobilized peripheral blood. In response to this problem, and as alternative sources to BM and MPB, HSCs from umbilical cord blood may be used for haematopoietic transplantation. This source of HSCs provides a series of important advantages compared with BM and MPB, such as the lesser degree of correspondence of the major histocompatibility complex of donor HLA - recipient necessary, the lower incidence of donor-versus-host disease, and the great availability of units stored in the cord banks of the whole world, which facilitates the rapid location of potential donors *(*Barker et al. Searching for unrelated donor haematopoietic stem cells: availability and speed of umbilical cord blood versus bone marrow. Biol Blood Marrow Transplant 2002;8:257-260*).*

The major disadvantage in the transplantation of UCB is the low cell dose of HSCs per unit available compared with those available in MPB and BM. This particular feature results in a greater risk of failure of the transplant and raised myeloid aplasia times, which leave the patient exposed to contracting potentially fatal infections. This problem limits the use of HSCs from UCB basically to paediatric transplantation. The quantity of HSCs available for transplantation is therefore a critical parameter. The best parameter for prediction of survival and recovery of the normal haematological parameters in paediatric and adult patients is the cell dose *(*Barker et al. Serious infections after unrelated donor transplantation in 136 children: impact of stem cell source. Biol Blood Marrow Transplant. 2005; 11:362-370*) .*

Various studies carried out with HSCs from MPB and BM have made it possible to establish a minimum dose required according to the cell source which was fixed at 2x10⁶ cells per kilo of patient in BM and 3-5x10⁶ cells per kilo of patient for MPB *(*Heimfeld et al. HLA-identical stem cell transplantation: is there an optimal CD34 cell dose?. Bone Marrow Transplantation 2003;31:839-845*).*

These results suggest that the methodologies for expanding the haematopoietic stem/parent cells of UCB would suppose a notable improvement in the available therapeutic alternatives. There are various promising strategies available for the in vitro expansion of HSCs with different systems *(*Cabrita et al. Haematopoietic stem cells from the bone to the reactor. Trends in Biotechnology 2003; 21:223-240*),* but at the moment in clinical trials carried out, these methodologies have not demonstrated outstanding improvements in haematological recoveries with respect to non-expanded UCB units.

The principal limitation of many of the *in vitro* expansions is the limited dose of stem/parent cells with adequate functionality which is obtained.

One of the principal problems of *in vitro* culturing of HSCs is the blocking of the growth capacity of said cells. This blocking occurs as a consequence of the accumulation in the culture medium of biomolecules released by the HSCs themselves and the mature cells present in the culture. These biomolecules have the effect of arresting the cycle of the HSCs, blocking the expansion or differentiation by causing them to become quiescent.

The present invention discloses a procedure for in vitro culturing of HSCs in which the strategy is based on maintaining the concentration of the soluble biomolecules responsible for the arresting of the growth of the HSCs in the GO/G1 phase below their level of action, on the basis of carrying out precise additions of fresh medium. In this way, the growth of HSCs from umbilical cord blood may be induced and maintained at an approximately constant pace for the entire duration of culturing.

This particular feature makes it possible to control and manipulate the expansion factor (final cells/initial cells) for the population of interest, which facilitates adapting said expansion factor according to the cell dose required for the therapy.

Another additional aspect of the procedure of the present invention is that said expansion does not take place at the expense of exhausting the sub-population of more primitive HSCs, which are of great therapeutic interest, since their number remains constant throughout the procedure. On the other hand, the procedure of the present invention, by varying the growth factors employed, makes it possible to effect maturing expansion of the HSCs, which makes it easy to obtain products intended for cell therapy based on cells in distinct stages of differentiation of myeloid lineages. Said cells are applicable to deficiencies of the immune system.

Other additional advantages are that the procedure is robust and reproducible and that it is extremely simple, both with regard to equipment and to handling. The procedure of the present invention is applicable to the undifferentiated or maturing expansion of HSC cells starting from umbilical cord blood, bone marrow or mobilized peripheral blood.

### DETAILED DESCRIPTION OF THE INVENTION

Consequently, it is an aim of the invention to disclose a procedure for the *in vitro* undifferentiated or myeloid lineage biased expansion of HSCs starting from umbilical cord blood, bone marrow or mobilized peripheral blood. This procedure is characterized in that it comprises the stages of:
a) Expansion at constant volume, in which CD34+ cells, previously purified by standard techniques known in the state of the art, such as positive selection by means of paramagnetic beads combined with antibodies against CD34+, are sown in a suitable commercially available synthetic culture medium, supplemented with growth factors: TPO, FLT3, SCF, IL-6 in the case of undifferentiated expansion of HSCs, or SCF, IL3, G-CSF in the case of maturing expansion to myeloid lineage, for 4 days. The initial volume of the constant volume stage depends on the availability of cells arising from the initial purification of the CD34+ cells. The final volume in the stage of expansion at constant volume will also depend on the initial sowing density.
b) Expansion at variable volume: on day 4 of culturing, fresh medium, supplemented with growth factors: TPO, FLT3, SCF and IL-6, in the case of undifferentiated expansion of HSCs, or SCF, IL-3 and G-CSF in the case of maturing expansion to myeloid lineage, is added to the culture bag at a concentration of between 5 and 100 ng/ml, until the previous volume contained in the bag is doubled. Said operation is repeated on days 6, 8, 10, 12, 14, 16 and 18. Culturing stops at 20 days.

Preferably, the concentration of CD34+ cells in the sowing of stage (a) is 100,000 to 1,000,000 CD34+ cells per ml of culture medium.

Also preferably, the concentration of growth factors TPO, FLT3, SCF, IL-6 in the culture medium is between 5 and 100 ng/ml.

### Case 1: Expansion of undifferentiated HSCs

In the case of applying the procedure described, using as growth factors FLT3, SCF and IL-6 with the aim of obtaining the undifferentiated expansion of HSCs, at 20 days of culturing, a rate of expansion of the HSC population of, at minimum, 200 times, is obtained, therefore producing a dose of undifferentiated cells which is suitable for their clinical use. If the treatment requires a larger number of cells, the procedure of doubling the volume may be repeated every 2 days until the necessary quantity of cells is obtained.

In addition, the HSCs obtained by the procedure of the present invention maintain the phenotype and the functionality characteristic of these cells: the number of units forming mixed colonies CFU-mix, the units forming BFU-E erythroid colonies, and the units forming CFU-GM granulocyte-macrophage colonies, is expanded. The number of cells with the capacity for forming colonies in the long term is maintained. On the other hand, the expanded cells showed a capacity for grafting in examples of immunodeficient rat (NOD-SCID). In addition, the product obtained is suitable for use in cell therapy from the point of view of biosecurity. The cells obtained do not exhibit chromosome changes and do not exhibit signs of induction of early apoptosis.

The procedure is robust, since the anticipated rate of expansion is obtained independently of the initial purity of the CD34+ cells. In addition, the procedure of the present invention has a high batch to batch reproducibility.

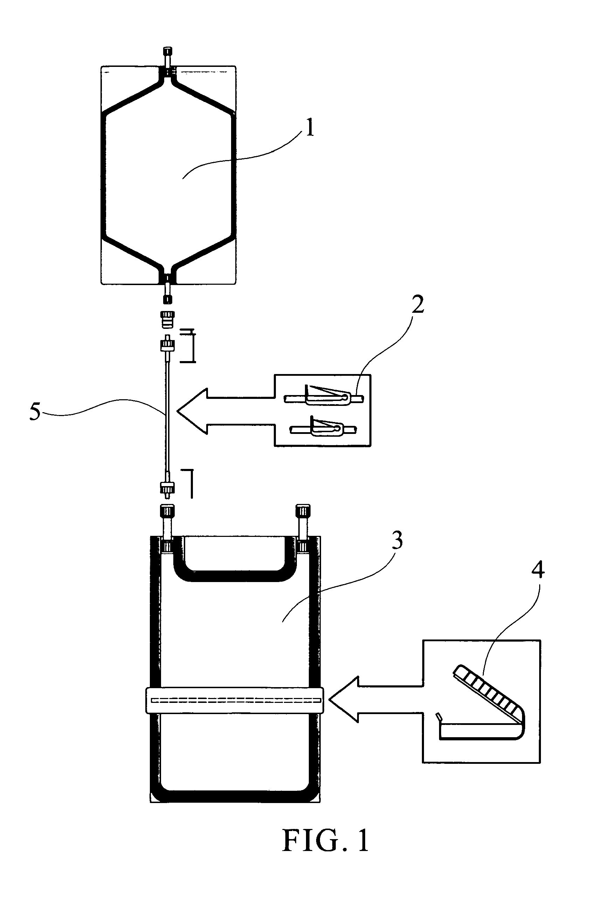

The present invention is described hereinafter in more detail with reference to an example and a drawing (Figure 1). This example, however, is not intended to limit the technical scope of the present invention.

### EXAMPLE 1

Figure 1 shows a system suitable for carrying out the procedure of the present invention. 500,000 CD34+ cells, which were previously defrosted and purified, were sown per ml in a gas-permeable Teflon bag 3 (bioreactor) which contained 50 ml of GMP commercial synthetic culture medium supplemented with growth factors TPO, FLT3, SCF and IL6 at a concentration of 50 ng/ml. Culturing was maintained for 4 days, during which time the concentration of cells increased to 800,000 CD34+ cells per ml. On day 4 the volume of the bag of culture 3 was doubled, increasing the capacity of the bag with the blocking clip 4. The fresh culture medium was stored in the storage bag 1 and was transferred to the culture bag by opening the clip 2 for regulating the flow volume through the connecting tube 5 to the culture bag 3 until the initial volume was doubled. This operation was repeated every two days until day 18. On day 20 culturing stopped.

An increase in the number of cells with the capacity for forming mixed colonies of 106 times, erythroid colonies 74 times and granulocyte/macrophage colonies 570 times was obtained. The number of cells with the capacity for forming colonies in the long term was kept substantially constant, which had an expansion rate of around 1. The expansion of the CD34+ cells was 200 times, while the expansion of the mononuclear cells was 2,000 times.

### Case 2: Myeloid lineage biased expansion of HSCs

In the case of applying the previously described procedure using as growth factors G-CSF, SCF and IL-3 with the aim of obtaining a maturing expansion of HSCs, at 20 days of culturing, a rate of expansion of the population of cells with the capacity for forming colonies (CFU) of approximately 1,600 times is obtained, therefore producing a cell dose suitable for clinical use. If the treatment requires a larger number of cells, the procedure of doubling the volume may be repeated every 2 days until the necessary quantity of cells is obtained.

The product obtained by the procedure of the present invention and using as growth factors IL-3, SCF, G-CSF consists of a heterogeneous cell combination with regard to maturing state within the haematopoietic myeloid lineage. The cells obtained exhibit, for the majority, a phenotype characterized by the combined expression of the markers CD34-, CD45+, CD11high CD15+ and a restriction of their multi-potent capacity to granulocyte/macrophage lineage. At the start of culturing, the percentage of units forming colonies of granulocyte/macrophage lineage supposes 35% of the whole of the population of cells with the capacity for forming colonies, while on completing the stage of maturing expansion this percentage increased to 91%. This datum shows the effectiveness of the procedure described in biasing the HSCs to myeloid/granulocyte lineage.

In addition, the product obtained is suitable for use in cell therapy with regard to biosecurity. The cells obtained do not exhibit signs of induction of early apoptosis.

The procedure is robust, since the anticipated expansion rate is obtained independently of the initial purity of the CD34+ cells. In addition, the procedure of the present invention has a high batch to batch reproducibility.

The present invention is described in more detail hereinafter with reference to an example and a drawing (Figure 1). This example, however, is not intended to limit the technical scope of the present invention.

### EXAMPLE 2

Figure 1 shows a system suitable for carrying out the procedure of the present invention. 10,000 CD34+ cells, which were previously defrosted and purified, were sown per ml in a gas-permeable Teflon bag 3 (bioreactor) which contained 5 ml of GMP commercial synthetic culture medium supplemented with growth factors IL3, SCF and G-CSF at a concentration of 50 ng/ml. Culturing was maintained for 4 days, during which time the concentration of cells increased to 50,000 CD34+ cells per ml. On day 4 the volume of the culture bag 3 was doubled, increasing the capacity of the bag with the blocking clip 4. The fresh culture medium was stored in the storage bag 1 and was transferred to the culture bag by opening the clip 2 for regulating the rate of flow through the connecting tube 5 to the culture bag 3 until the initial volume was doubled. This operation was repeated every two days until day 18. On day 20 culturing stopped.

An increase in the number of cells with the capacity for forming colonies of granulocytes/macrophages of 1,600 times was obtained. The expansion of the CD34+ cells was 200 times, while the expansion of the mononuclear cells was 2,000 times.

Although the invention has been described with respect to the preceding examples, these should not be regarded as limiting the invention, which will be defined by the widest interpretation of the following claims.

## Claims

1. A procedure for the undifferentiated or myeloid lineage biased expansion of haematopoietic stem cells from umbilical cord blood, mobilized peripheral blood or bone marrow, **characterized in that** it comprises the stages of:
a) Expansion at constant volume, in which CD34+ cells, previously purified by standard techniques known in the state of the art, such as positive selection by means of paramagnetic beads combined with antibodies against CD34+, are sown in a suitable commercially available synthetic culture medium, supplemented with growth factors: TPO, FLT3, SCF, IL-6 in the case of undifferentiated expansion of HSCs, or SCF, IL3, G-CSF in the case of maturing expansion to myeloid lineage, for 4 days. The initial volume of the constant volume stage depends on the availability of cells arising from the initial purification of the CD34+ cells. The final volume in the stage of expansion at constant volume will also depend on the density of the initial sowing.
b) Expansion at variable volume: on day 4 of culturing, fresh medium, supplemented by growth factors: TPO, FLT3, SCF and IL-6, in the case of undifferentiated expansion of HSCs, or SCF, IL-3 and G-CSF in the case of maturing expansion to myeloid lineage, is added to the culture bag at a concentration of between 5 and 100 ng/ml until the previous volume contained in the bag is doubled. Said operation is repeated on days 6, 8, 10, 12, 14, 16 and 18. Culturing stops at 20 days.

2. A procedure according to claim 1, wherein in the stage of expansion at constant volume 100,000 to 1,000,000 CD34+ cells are sown per ml of culture medium.

3. A procedure according to claims 1 and 2, wherein the concentration of growth factors TPO, FLT3, SCF, IL-6 in the culture medium is between 5 and 100 ng/ml.

4. A procedure according to any one of the preceding claims, wherein at 20 days of culturing the rate of expansion of the CD34+ population is, at minimum, 200 times.
